# EUROPEAN PATENT APPLICATION

(11) **EP 4 574 099 A1**
(43) Date of publication of application: **25.06.2025**
(21) Application number: 23220053.5
(22) Date of filing: 22.12.2023
(51) Int. Cl.: A61F 2/50, A61F 2/76, A61F 2/80

(54) **METHOD FOR MANUFACTURING A NEW PROSTHESIS**

(71) Applicant: Ottobock SE & Co. KGaA, 37115 Duderstadt (DE)
(72) Inventor: DAUR, Christian, 37115 Duderstadt (DE); MAIER, Uli, 37115 Duderstadt (DE); MOUROUM, Marvin, 37115 Duderstadt (DE); RACH, Olaf, 37115 Duderstadt (DE); REYNOLDS, Dave, 37115 Duderstadt (DE); KHOUNLAVONG, Anousack, 37115 Duderstadt (DE); LONGUEVILLE, Matthieu, 37115 Duderstadt (DE); FONTAINE, Vincent, 37115 Duderstadt (DE); COCARDON, Xavier, 37115 Duderstadt (DE); REVAIS, Jules, 37115 Duderstadt (DE); GENTIT, Theo, 37115 Duderstadt (DE)
(74) Representative: Gramm, Lins & Partner Patent- und Rechtsanwälte PartGmbB

(57) **Abstract**

The Invention refers to a method for manufacturing a new prosthesis for a lower limb, the method comprising the following steps:
- a) Providing 3D-data of a prior inner surface of a prior socket and of ist position and its orientation relative to a component adapter, - b) Creating a 3D model of a prosthetic socket with an attachment area for a socket adapter, wherein the prosthetic socket has an inner surface based on the scanned prior inner surface and an outer surface, - c) Determining information about an orientation transmitter, - d) Manufacturing of the prosthetic socket based on the 3D model and providing the orientation transmitter based on the information, - e) Directly or indirectly connecting the manufactured socket to a component adapter by means of the orientation transmitter so that the position and orientation of the inner surface of the prosthetic socket relative to the position and orientation of the component adapter corresponds to the position and orientation of the prior inner surface of the prior socket to the component adapter.

## Description

The invention refers to a method for manufacturing a new prosthesis for a lower limb. The invention further refers to an orienter used in such a method and to a system manufactured during such a method.

Today, prostheses for a lower limb are known from prior art for a long time. They can be passive prostheses having no motor or external energy source or they can be active and have at least one joint or actuator that is driven by some external force or energy. No matter how the prosthesis is designed, they all have to be connected to the remainder of the lower leg, usually referred to as the amputation stump. In order to do so, a prosthesis for a lower limb comprises a prosthetic socket, having an open proximal end and a closed distal end. The amputation stump is inserted into the open proximal end and thereby enters a cavity inside the socket.

The socket is usually made from a rigid material, such as carbon or it is printed on a 3D printer. The material and the structural design of the socket has to withstand the forces and loads that occur during walking, running and/or jumping over a time period of several years. This is especially challenging since the prosthesis and thus the socket is usually worn every day for several hours, if not for the whole day. Thus the socket is subject to wear and it has to be renewed and replaced after some time. The socket also has to be replaced by a new socket when the amputation stump changes, for example by losing or gaining volume or by achieving new levels of mobility, for example by therapy or training.

In all these cases the socket has to be replaced. This is a difficult task, since the socket is not used on its own, but as a part of a system configured from several prosthetic parts, which usually are carefully aligned relative to one another. For example, the position and orientation of the socket relative to a prosthetic foot is crucial for a good fit, a high comfort and a high functionality of the prosthetic device. If for example the distance between the prosthetic socket and the prosthetic foot is too large or to small the prosthetic device is too long or too short for the wearer thus preventing a comfortable fit and a naturally looking walking pattern. Not only the length of the prosthetic device is crucial for a comfortable fit and walking experience but also the orientation and position of different components of the prosthetic device relative to each other. In order to achieve a good setup of the prosthetic device the wearer has to wear the prosthesis and perform several tasks like standing still, walking along a plane or a slope and climb or descend stairs. The user is watched or even filmed and the results are then used to improve the setup of the prosthesis. This can be done in several iterations until a satisfying result is achieved.

When the new socket is produced one has to redo all this in order to get the new socket and the new prosthesis as well arranged and setup as the prior prosthesis. This is time consuming and uncomfortable for the wearer.

Another challenge occurs when fitting a patient with a socket for the first time. Usually, in this case a so called check socket, which is a provisional socket, is created. The shape of this check socket is modified until it fits best to the patient. The check socket thus need to be reshapable in contrast to the definitive socket, which is then to be manufactured and used by the patient over a long time. It is desirable to capture the modifications and adjustments made to the check socket as well as the alignment of the socket relative to other components of the prosthesis. This prosthetic alignment is already done and established by the orthopedic technician using the check socket. It is thus desirable to also transfer this check socket alignment to the definitive socket instead of having to redo the entire alignment procedure.

In other situations a prosthetic socket might be purely digital including the alignment of the entire prosthesis. While there are existing software tools available to perform a virtual bench alignment of the prosthesis, with the methods known from prior art, it is not possible to transfer the digitally determined and/or verified alignment to a physical "real" prosthesis. The alignment has to be redone from the start once the prosthetic socket is manufactured from the digital model and data or at least the digitally provided alignment has to be reproduced step by step by copying each setting of the virtual alignment. This is time consuming both for the orthopedic technician and the patient. Also the result of the alignment procedure might deviate from the desired and digitally verified alignment goal.

In all of these situations and cases there is a need for transferring a digitally available alignment of at least a part of a prosthetic socket to another element of the prosthesis into the "real" world and onto the manufactured prosthesis. In all of these situations and cases a "prior socket" is the starting point. This prior socket can be an earlier definitive socket that is to be replaced. It can also be a check socket that is used to find the best fit for a definitive socket. The prior socket can also be a socket, that exists only digitally in form of some model or a set of data. From all of these different forms of "prior socket" a new socket is to be manufactured and the alignment information known for the prior socket is to be transferred to the prosthesis a part of which the new socket is to be.

It is thus a task for the present invention to provide a method to manufacture a new prosthesis for a lower limb that prevents or at least mitigate the disadvantages from prior art.

The task is solved by a method for manufacturing a new prosthesis for a lower limb, the method comprising the following steps:
a) Providing 3D-data of a prior inner surface of a prior socket and of its orientation relative to a component adapter,
b) Creating a 3D model of a prosthetic socket with an attachment area for a socket adapter, wherein the prosthetic socket has an inner surface based on the provided 3D-data of the prior inner surface and an outer surface,
c) Determining information of an orientation transmitter,
d) Manufacturing of the prosthetic socket based on the 3D model and providing the orientation transmitter based on the information, and
e) Directly or indirectly connecting the manufactured socket to a component adapter by means of the orientation transmitter so that the position and orientation of the inner surface of the prosthetic socket relative to the position and orientation of the component adapter corresponds to the position and orientation of the prior inner surface of the prior socket to the component adapter.

No matter what kind of prior socket is used, whether it is based on an already existing definitive socket or check socket or whether it only exists digitally, it always comprises a prior inner surface. This is the surface oriented towards the amputation stump when the prior socket is worn and an amputation stump is placed in the cavity of the prior socket. The already existing or digitally planned prosthesis comprises a prosthetic component, such as a prosthetic foot or a prosthetic joint. The component comprises a component adapter to connect the component to other elements of the prosthesis such as a lower leg tube, which is often simply called "tube", or the prior socket. The other element then preferably comprises a connector that is arranged and adapted to be connected to an adapter, such as the component adapter. When a tube is used, the tube usually has a connector on both ends, one of which is to be connected to the component adapter of the prosthetic component and the other one is to be connected to a socket adapter provided on the outer surface of the respective socket. If the prior socket has a socket adapter, the new socket also is to be equipped with a socket adapter. In order to be able to attach the socket adapter to the socket, there is the attachment area. Preferably the socket adapter can be positioned and attached to the prosthetic socket in the attachment area in only very specific orientations.

In some embodiments of the present invention only the prosthetic socket is to be replaced. This means, that the only element distinguishing the new prosthesis from the prior prosthesis is the prosthetic socket. The prior prosthesis comprises the prior socket and the new prosthesis comprises the new prosthetic socket, also referred to as "prosthetic socket". In other embodiments of the present invention further elements of the prosthesis are replaced, such as the component adapter and/or the tube.

If the prior socket only exists digitally, then of course no components can be reused in the prosthesis that is to be manufactured. In this case the prior socket and its alignment to other components of a prosthesis exist only digitally wherein the alignment often is digitally verified. This is then to be transferred to the real world prosthesis via manufacturing at least part of the prosthesis and transferring the alignment via the orientation transmitter.

First, 3D-data of the prior inner surface of the prior socket and of its position and orientation relative to the component adapter is provided. It is not necessary to also provide 3D-data of the prior outer surface of the prior socket or of any other element or component of the prior prosthesis. The 3D-data advantageously are stored in a digital form in a data storage device accessible for an electronic data processing device, such as a computer. In some embodiments it can be useful to modify the position and/or orientation in a virtual alignment software, for example if some of the prosthetic components are to be changed.

This computer is then used to create a 3D model of the new prosthetic socket, that is to replace the prior socket. A person skilled in the art knows several ways to do so, for example by taking into account simulation results of simulations in which the expected loads, forces and torques are simulated. Since it is not necessary to know the prior outer surface of the prior socket, the outer surface of the prosthetic socket can be very different from the prior outer surface in structure and/or shape. Also, the position and orientation of the socket adapter relative to the inner surface of the prosthetic socket can be different from the position and orientation of the prior socket adapter relative to the prior inner surface. The 3D-data of the prior inner surface are preferably adapted and manipulated before the inner surface and the corresponding 3D model is created.

The 3D-data of the prior inner surface of the prior socket can stem from an inner scan of a physical socket, such as an already existing definitive socket or a check socket, in which case little to no modifications of the 3D-data is necessary. The 3D-data can also be derived from a scan of the amputation stump in which case the surface of the amputation stump corresponds to the prior inner surface. In this case more modifications of the 3D-data are necessary.

The computer is also used to determine information of an orientation transmitter. In a preferred embodiment the computer in this method step creates a 3D model of the orientation transmitter. This is created based on the information concerning the position and orientation of the prior inner surface relative to the component adapter which has to be the same as the position and the orientation of the inner surface of the prosthetic socket relative to the component adapter in the new prosthesis.

Then the prosthetic socket is manufactured based on the created 3D model and the orientation transmitter is provided based on the information determined. In some embodiments the orientation transmitters are separate elements that can be manufactured individually or that can be chosen from an existing set of orientation transmitters. The computer determines information of the orientation transmitter that allows to choose the desired orientation transmitter that is to be used in the respective method. In some embodiments the orientation transmitter might be adjustable in which case the determined information also allows to adjust the orientation transmitter as necessary. In other embodiments the computer creates a 3D model of the orientation transmitter. The orientation transmitter is then manufactured based on this 3D model.

Then the new prosthesis is assembled. Therefore, the manufactured socket is directly or indirectly connected to the component adapter of the prosthesis, which can be the component adapter that has already been used in the prior prosthesis or it can be a new one. The socket is connected to the component adapter by means of the orientation transmitter, so that the correct orientation and position of the inner surface of the prosthetic socket relative to the component adapter is obtained. Due to this, it is not necessary to perform a complete setup for the new prosthesis, but the wearer can use the new prosthesis as the prior one, without any additional adjustments. Using the orientation transmitter it becomes possible to transmit the position and orientation of the prior inner surface relative to the component adapter of the prior prosthesis to the new prosthesis.

In a preferred embodiment method step a) comprises the following steps:
a1) Providing a prior prosthesis for the lower limb, the prior prosthesis comprising the prior socket and a prior connector for connecting to a component adapter of a prosthetic component in a predetermined prior orientation and
a2) Scanning the prior inner surface of the prior socket and detecting its position and orientation relative to the position of the component adapter.

The prior connector can be arranged at one end of a prior tube, that indirectly connects the prior socket to the component adapter. The prior connector can also be arranged at the prior outer surface of the prior socket. It is possible and in some embodiments advantageous to scan the prior inner surface using a handheld or a static scanning device. Preferably the position and orientation of the prior inner surface relative to the component adapter can be detected from the scan-data gathered during the scanning.

Preferably the prior connector comprises four screws and the component adapter is a pyramid adapter known in the prior art for a long time. The method step a1) in this case preferably comprises the following steps:
a1 i) Unscrewing two neighboring screws of the prior connector,
a1 ii) Removing the prior connector from the component adapter,
a1 iii) Positioning the prior prosthesis with the prior connector on a holding pyramid adapter
a1 iv) Tightening the two unscrewed screws.

With these steps, the orientation in which the prior connector was positioned and arranged on the component adapter is preserved and transmitted, so that after performing these steps, the prior connector is arranged on the holding pyramid adapter in the same orientation as it was on the component adapter. The holding pyramid adapter is preferably a part of a holding device. Once the prior connector is connected to the holding pyramid adapter and thus to the holding device, the prior inner surface is scanned. During this scan at least one, preferably at least two, more preferably at least three reference marks are also scanned, which are part of or at least positioned on the holding device. Since the position of the holding pyramid adapter relative to these marks is known and the scan-data show both the prior inner surface and the marks, one can easily compute the position and orientation of the prior inner surface relative to the holding pyramid adapter.

In some embodiments method step e) comprises connecting a tube having a connector at each of its ends to a socket adapter arranged on the manufactured prosthetic socket and/or to the component adapter.

Preferably the tube is provided using the following steps:
- Providing a tube having a connector at its first end,
- Installing a guide tool on the provided tube and aligning it with the connector on the first end,
- Shifting a measuring element along the guide tool to a desired position, wherein the measuring element has a circumferential marking line and an axial marking line,
- Marking the position of the measuring element relative to the tube using the circumferential marking line and marking the orientation of the measuring element relative to the tube using the axial marking line,
- Cutting the tube according to the marked position and
- Arrange a connector at the second end of the tube according to the marked orientation.

Since the outer surface of the prosthetic socket is not necessarily identical to the prior outer surface of the prior socket, also the position of the socket adapter may have changed. This means, that a tube used for the prior prosthesis might be too long or too short for the new prosthesis. With the steps according to this embodiment, it is possible to manufacture a new tube having the needed length.

First, a tube is provided that has a first connector on its first end and that is longer than needed. The first connector preferably comprises four screws to connect it to a pyramid adapter. These four screws can easily be used to align the guide tool with the connector. A measuring element, capable of sliding along the guide tool is then brought into the desired position by shifting it along the guide tool. The measuring tool has a circumferential marking line, which for example can be its rim. One of the easiest measuring element is a ring having a small slit. The rim of the ring serves as the circumferential marking line and the slit serves as the axial marking line. It is advantageous, when the slit extends in an axial direction. Preferably this direction is parallel to the shifting direction.

Once the ring is in the desired position one uses the circumferential line to mark this position and one uses the slit as the axial marking line to mark the orientation. With these two markings it is then possible to manufacture the tube by cutting it corresponding to the marked position so that the tube gets the correct length. Then one arranges the second connector to the second end of the tube corresponding to the marked orientation. Preferably the two connectors are oriented aligned to one another. The desired tube length and orientation are determined from the orientation of the prior inner surface and either provided to the technician or the ring can be manufactured individually and the desired specifications can be intrinsically incorporated into its design, preferably by manufacturing the axial slit at the correct position.

In some embodiments the prosthetic socket and/or the orientation transmitter are manufactured in method step d) at least partially using an additive manufacturing process such as 3D printing.

In some embodiments of the present invention the orientation transmitter comprises a plurality of markings applied to or in the outer surface of the prosthetic socket, wherein the prosthetic socket is placed in a holding device, wherein the prosthetic socket is oriented by means of the holding device by means of the marking. The holding device can be the same holding device that has also been used during scanning the prior inner surface of the prior socket. Of course, also different holding devices can be used. The holding device preferably also comprises a holding adapter, to which the prosthetic socket is to be directly or indirectly coupled. The holding device can furthermore include menas for holding and/or moving the prosthetic socket during the transfer of the alignment.

The holding device comprises an aligning device, such as laser beams, that can be used to align the markings of the orientation transmitter with the holding device. Once this alignment is completed the prosthetic socket is known to be in the correct position and orientation relative to the holding device and thus also relative to the holding adapter. Then the prosthetic socket is connected to the holding adapter directly or indirectly. The holding adapter is then connected to a connector, which preferably comprises four screws. The holding adapter preferably is a pyramid adapter. After connecting the connector to the holding adapter, one unscrews two neighboring screws, removes the prosthesis from the holding adapter, arranges the connector on a component adapter of a component and tightens the two screws. In this way, the orientation, in which the connector was connected to the holding adapter is preserved and is identical to the orientation, in which the connector is connected to the component adapter.

In some embodiments the orientation transmitter comprises at least four, preferably at least six markings, by means of which the prosthetic socket is aligned in three spatially independent directions relative to the holding device.

In some embodiments the orientation transmitter comprises a socket orienter and/or a component orienter, each of which has a contact edge and an inverted pyramid, the contact edge being annual, preferably circular, and the inverted pyramid being tilted relative to the plane defined by the contact edge by a predetermined angle in a predetermined direction. The invers pyramid corresponds in size and shape to the inverted pyramid of a pyramid adapter, but compared to this is tilted as a whole.

Preferably connecting the tube with the socket adapter comprises the following steps:
- Positioning the tube so that the connector at one end of the tube rests against the contact edge of the socket orienter and the inverse pyramid of the socket orienter protrudes into the connector,
- Tightening the four screws of the connector at the end of the tube resting against the contact edge,
- Unscrewing two neighboring screws of the connector,
- Removing the tube from the socket orienter and positioning the end of the tube on the socket adapter and
- Tightening the two unscrewed screws.

Preferably connecting the tube with the component adapter comprises the following steps:
- Positioning the tube so that the connector at one end of the tube rests against the contact edge of the component orienter and the inverse pyramid of the component orienter protrudes into the connector,
- Tightening the four screws of the connector at the end of the tube resting against the contact edge,
- Unscrewing two neighboring screws of the connector,
- Removing the tube from the component orienter and positioning the end of the tube on the component adapter and
- Tightening the two unscrewed screws.

The socket orienter and the component orienter each have a diameter which is larger than the diameter of the tube. The contact egde protrudes radially so that the end of the tube with the connector arranged on this end rests against this contact edge. Radially outside thereof the contact edge is surrounded by a raised section that protrudes in axial direction. This raised section can be described as a ring-shaped rampart. It comprises at least one, preferably four recesses, which are used to orient the tube relative to the orienter. In order to achieve this, the tube and its connector are to be rotated around the longitudinal axis of the tube until the screws of the connector protrude into the recesses. This is done for both the socket orienter and the component orienter. The orienter preferably has an orientation marking defining the front side of the orienter. In preferred embodiments this is true for both orienters. It is advantageous to tighten all screws with the same predefined torque to avoid any displacements due to excessive or unbalanced forces applied by the screws on the material of the orienter.

In some embodiments the orientation transmitter is manufactured at the time as the prosthetic socket.

A method for manufacturing a tube usable in a method described above is a separate invention. According to this method the tube is provided using the following steps:
- Providing a tube having a connector at its first end,
- Installing a guide tool on the provided tube and aligning it with the connector at the first end,
- Shifting a measuring element along the guide tool to a desired position, wherein the measuring element has a circumferential marking line and an axial marking line,
- Marking the position of the measuring element relative to the tube using the circumferential marking line and marking the orientation relative to the tube using the axial marking line,
- Cutting the tube according to the marked position and
   Arrange a connector at the second end of the tube according to the marked orientation.

A tool usable in such a method is also an invention of its own. The tool comprises
- a guide tool with an alignment element to be aligned with a connector at one end of a tube,
- a measuring element, that is connected or connectable to the guide tool such that it can be shifted along the guide tool,
wherein the measuring element has a circumferential marking line and an axial marking line.

The invention solves the task in addition by an orienter for a method described above. It comprises a contact edge and an inverted pyramid, the contact edge being annular, preferably circular, and the inverted pyramid being tilted relative to the plane defined by the contact edge by predetermined angle in a predetermined direction, wherein preferably the angle is not 0°. The angle is preferably calculated from the 3D models of the prosthetic socket and the orientation transmitter and the 3D data of the position and orientation of the inner surface of the prosthetic socket relative to the component adapter.

The invention solves the task in addition by a system comprising a prosthetic socket and at least a first orienter. Both the prosthetic socket and the first orienter are preferably manufactured during a method described above. The system preferably further comprises at least a second orienter, wherein the first orienter is a socket orienter and the second orienter is a component orienter. In a preferred embodiment the system further comprises a tube having a connector at each of its ends. Alternatively, the system comprises a tube having a connnector at only one of its ends and a guide tool with measuring element as described above. The measuring element can comprise a circumferential marking line and an axial marking line.

The invention solves the task in addition by a method for manufacturing a system as described above, the method comprising the following steps:
- Providing 3D-data of a prior inner surface of a prior socket and of its position and its orientation relative to a component adapter,
- Creating a 3D model of a prosthetic socket with a socket adapter, wherein the prosthetic socket has an inner surface based on the scanned prior inner surface and an outer surface,
- Creating a 3D model of an orientation transmitter,
- Manufacturing of the prosthetic socket and the orientation transmitter based on the 3D models
wherein the orientation transmitter is created such that the manufactured socket can be directly or indirectly connected to a component adapter by means of the orientation transmitter so that the position and orientation of the inner surface of the prosthetic socket relative to the position and orientation of the component adapter corresponds to the position and orientation of the prior inner surface of the prior socket to the component adapter.

By means of the accompanying drawings, some embodiments are further described.
- Figures 1 to 6: show different stages during a method according to an embodiment of the present invention,
- Figures 7 to 10: show different stages during a method to produce a tube according to one embodiment of the present invention
- Figures 11 and 12: show an orienter in different views,
- Figure 13: shows two different orienters in a schematic sectional view,
- Figure 14: shows a tube with a connector connected to an orienter,
- Figure 15: shows schematically a step of the method,
- Figure 16: shows a flow chart combining several embodiments of the present invention and
- Figure 17: shows a schematic sectional view of a connector positioned at an orienter.

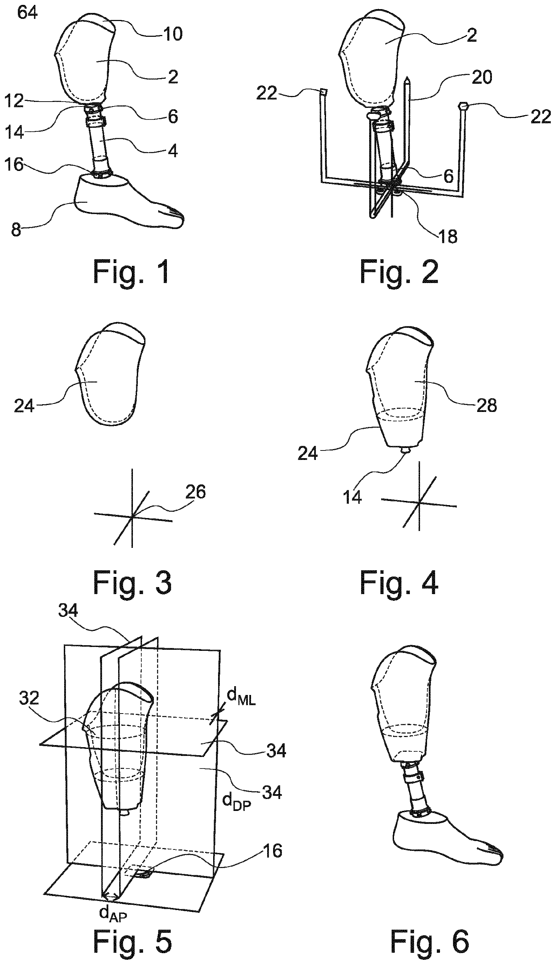

Figure 1 shows a prior prosthesis having a prior socket 2, a tube 4, which has a connector 6 at each of its ends, and a component 8 in form of a prosthetic foot. The prior socket 2 comprises an open proximal end 10 and a closed distal end 12 to which a socket adapter 14 is mounted. The socket adapter 4 is connected to one of the connectors 6. The component 8 comprises a component adapter 16. The prior socket 2 is to be replaced by a new socket.

Figure 2 shows the prior prosthesis, without the component. The connector 6, that was connected to the component adapter is now mounted to a holding pyramid adapter 18, which is part of a holding device 20. The holding device 20 comprises four reference marks 22, the position of which relative to the holding pyramid adapter 18 is known. In this arrangement the prior inner surface of the prior socket 2 is scanned. During this scanning also at least some of the reference marks 22 are scanned.

A schematic sketch of the scan-data is shown in Figure 3. One can see the scan-data 24 corresponding to the prior inner surface and the position 26 of the holding pyramid adapter relative to the prior inner surface.

Figure 4 shows 3D model 28 of the new prosthetic socket, having a newly constructed outer surface 30 and a socket adapter 14. In Figure 5 the manufactured prosthetic socket 32 is shown. It is aligned with planes 34 using markings on the outer surface of the prosthetic socket. Three different offsets d_{ML}, d_{DP} and d_{AP} are shown, unambiguously defining the position and the orientation of the prosthetic socket relative to the position of the component adapter 16. In Figure 6 the new prosthesis is shown. It has the same orientation and position of the inner surface of the socket relative to the component, but looks different from the prior prosthesis shown in Figure 1.

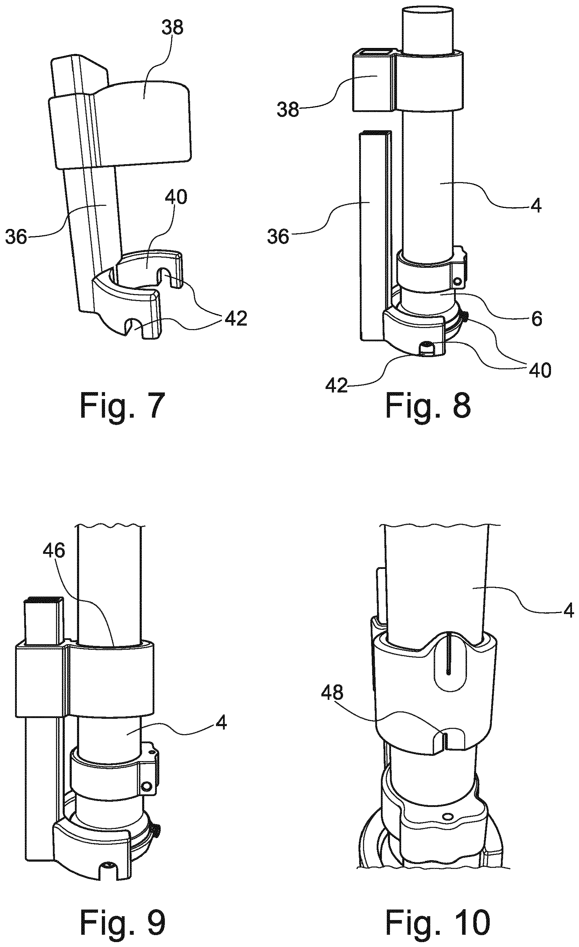

Figure 7 shows a guide tool 36 with a measuring element 38, capable of sliding up and down the guide tool 36. At the lower end of the guide tool 36 there is an alignment element 40 heaving two recesses 42, which can be used to align the guide tool 36 with a connector on one end of a tube. In Figure 8 a tube 4 is positioned in the guide tool 36. At the lower end of the tube 4 there is a connector 6 with four screws 44, one if which is shown to protrude through one of the recesses 42. The measuring element 38 has not yet been arranged on the guide tool 36.

In Figure 9 the measuring element 38 has been shifted into the desired position. It comprises a circumferential marking line 46, which in Figure 9 is used to mark the position of the measuring element 38 on the tube 4. In Figure 10 it can be seen, that the measuring element 38 also has a axial marking line 48, which is used to mark the orientation of the measuring element 38 on the tube 4.

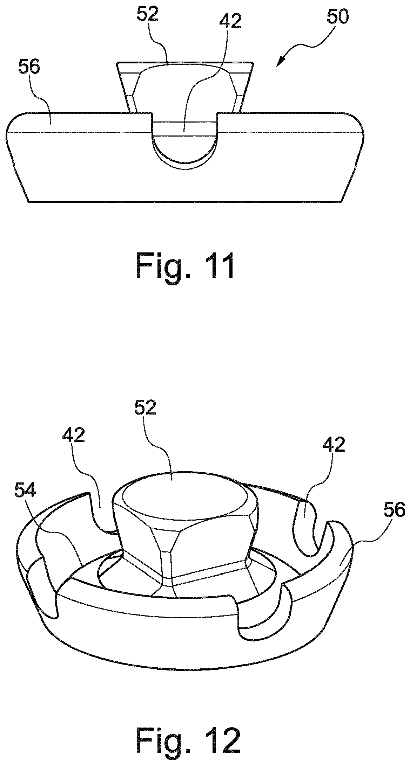

Figures 11 and 12 show an orienter 50. It has an inverted pyramid 52, having four sides. It is surrounded by a contact edge 54, which is surrounded by a raised section 56 having four recesses 42.

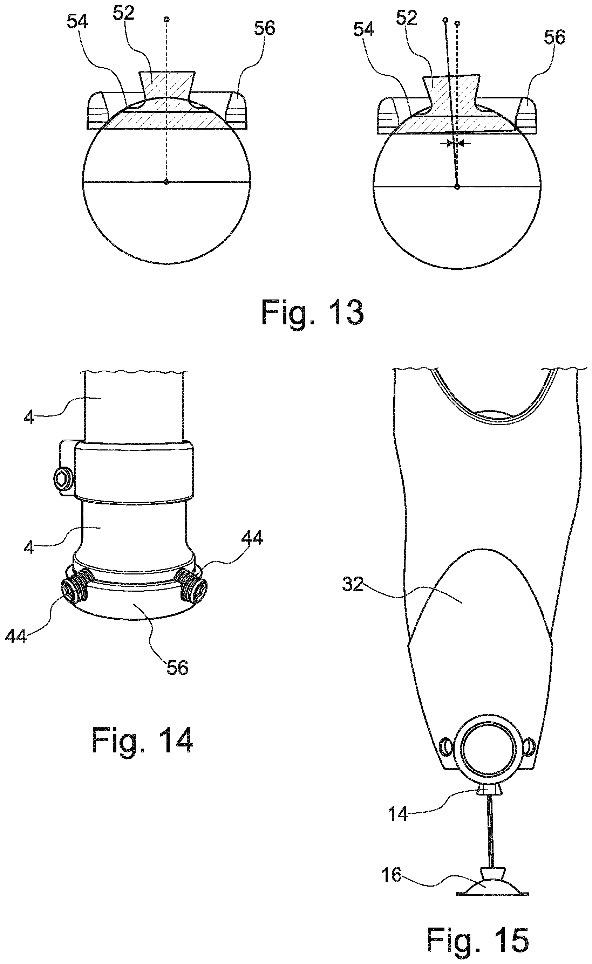

In Figure 13 two different orienter 50 are shown. They both have the inverted pyramid 52, with the right inverted pyramid being tilted by a small angle compared to the left one. The contact edge 54 in both cases lies on a sphere and the inverted pyramids 52 are tilted relative to one another by a rotation around the center of this sphere.

Figure 14 shows the end of a tube 4 with a connector 6 at its end. The connector 6 has four screws 44, two of which are shown in Figure 14. They are positioned in the recesses 42 of the raised section 56 of the orienter 50.

Figure 15 shows schematically that after creating the 3D model of the prosthetic socket 32, the socket adapter 14 and the component adapter 16 are also modelled in order to determine information about the orientation transmitter.

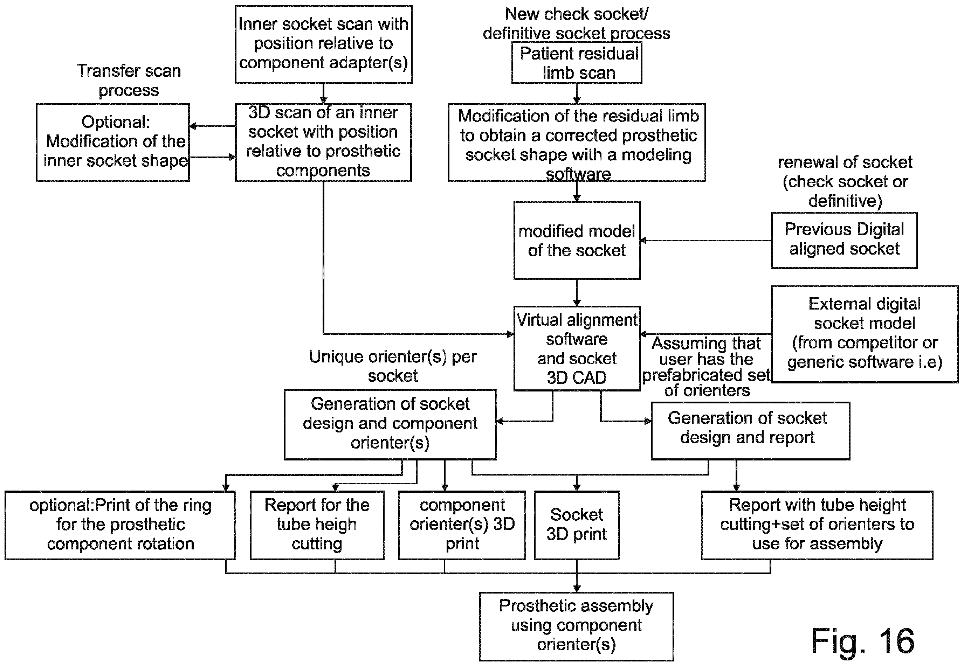

Figure 16 sums up a number of different embodiments of the present invention and is self explanatory.

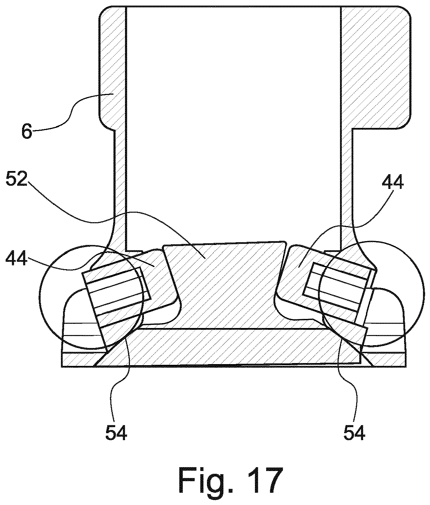

In Figure 17 a part of a schematic sectional view is shown showing a connector 6 which is positioned at an orienter, which could be a component orienter or a socket orienter. At the lower end of the connector there are four screws 44, two of which are shown in Figure 17. The orienter has the inverted pyramid 52, which in Figure 17 is slightly tilted to the left. Each of the screws 44 contact one side of the inverted pyramid 52, when the connector 6 is mounted to the adapter as it is shown in Figure 17. Once two neighboring screws 44 get unscrewed, the connector 6 can be removed from the orienter, but the orientation between the connector 6 and the adapter is preserved in the position of the two screws 44 that were not unscrewed. This can be used to transfer this orientation when the connector 6 gets connected to an adapter.

### List of reference numbers:

2 prior socket
4 tube
6 connector
8 component
10 proximal end
12 distal end
14 socket adapter
16 component adapter
18 holding pyramid adapter
20 holding device
22 reference mark
24 scan data
26 position
28 3D model
30 outer surface
32 prosthetic socket
34 plane
36 guide tool
38 measuring element
40 adjustment element
42 recess
44 screw
46 circumferential marking line
48 axial marking line
50 orienter
52 inverted pyramid
54 contact edge
56 raised section

## Claims

1. Method for manufacturing a new prosthesis for a lower limb, the method comprising the following steps:
- a) Providing 3D-data of a prior inner surface of a prior socket and of its position- and its orientation relative to a component adapter,
- b) Creating a 3D model of a prosthetic socket with an attachment area for a socket adapter, wherein the prosthetic socket has an inner surface based on the scanned prior inner surface and an outer surface,
- c) Determining information about an orientation transmitter,
- d) Manufacturing of the prosthetic socket based on the 3D model and providing the orientation transmitter based on the information,
- e) Directly or indirectly connecting the manufactured socket to a component adapter by means of the orientation transmitter so that the position and orientation of the inner surface of the prosthetic socket relative to the position and orientation of the component adapter corresponds to the position and orientation of the prior inner surface of the prior socket to the component adapter.

2. Method according to claim 1, wherein in method step c) a 3D model of the orientation transmitter is created and in method step d) the orientation transmitter is manufactured.

3. Method according to claim 1 or 2, wherein method step a) comprises
a1) Providing a prior prosthesis for the lower limb, the prior prosthesis comprising the prior socket and a prior connector for connecting to a component adapter of a prosthetic component in a predetermined prior orientation,
a2) Scanning the prior inner surface of the prior socket and detecting its position and orientation relative to the position of the component adapter.

4. Method according to claim 3, wherein the prior connector comprises four screws and the component adapter is a pyramid adapter, wherein method step a1) comprises the following steps:
a1 i) Unscrewing two neighboring screws of the prior connector,
a1 ii) Removing the prior connector from the component adapter,
a1 iii) Positioning the prior prosthesis with the prior connector on a holding pyramid adapter,
a1 iv) Tightening the two unscrewed screws.

5. Method according to any of the preceding claims, wherein method step e) comprises connecting a tube having a connector at each end to a socket adapter disposed on the manufactured prosthetic socket and/or to the component adapter.

6. Method according to any of the preceding claims, wherein the prosthetic socket and/or the orientation transmitter are manufactured in method step d) at least partially using an additive manufacturing process.

7. Method according to any of the preceding claims, wherein the orientation transmitter comprises a plurality of markings applied to or in the outer surface of the prosthetic socket, wherein the manufactured prosthetic socket is placed in a holding device to connect it to the component adapter, wherein the prosthetic socket is oriented relative to the holding device by means of the markings.

8. Method according to claim 7, wherein the orientation transmitter comprises at least four, preferably at least six markings, by means of which the prosthetic socket is aligned in three independent spatial directions relative to the holding device.

9. Method according to claim 7 or 8, wherein for connecting the tube to the component adapter, the component adapter is arranged in a predetermined orientation and position relative to the holding device.

10. Method according to any of the preceding claims 1 to 6, wherein the orientation transmitter comprises a socket orienter and/or a component orienter, each of which has a contact edge and an inverted pyramid, the contact edge being annular, preferably circular, and the inverted pyramid being tilted relative to the plane defined by the contact edge by a predetermined angle in a predetermined direction.

11. Method according to claim 10, wherein connecting the tube with the socket adapter comprises the following steps:
- Positioning the tube so that the connector at one end of the tube rests against the contact edge of the socket orienter and the inverse pyramid of the socket orienter protrudes into the connector,
- Tightening the four screws of the connector at the end of the tube,
- Unscrewing two neighboring screws of the connector,
- Removing the tube from the socket orienter and positioning the end of the tube on the socket adapter,
- Tightening the two unscrewed screws.

12. Method according to claim 10 or 11, wherein connecting the tube with the component adapter comprises the following steps:
- Positioning the tube so that the connector at one end of the tube rests against the contact edge of the component orienter and the inverse pyramid of the component orienter protrudes into the connector,
- Tightening the four screws of the connector at the end of the tube,
- Unscrewing two neighboring screws of the connector,
- Removing the tube from the component orienter and positioning the end of the tube on the component adapter,
- Tightening the two unscrewed screws.

13. Method according to any of claims 10 to 12, wherein the orientation transmitter is manufactured at the same time as the prosthetic socket.

14. Method for providing a tube usable in a method according to claim 4, wherein the tube is provided using the following steps:
- Providing a tube having a connector at its first end,
- Installing a guide tool on the provided tube and aligning it with the connector at the first end,
- Shifting a measuring element along the guide tool to a desired position, wherein the measuring element has a circumferential marking line and an axial marking line,
- Marking the position of the measuring element relative to the tube using the circumferential marking line and marking the orientation relative to the tube using the axial marking line,
- Cutting the tube according to the marked position
- Arrange a connector at the second end of the tube according to the marked orientation.

15. Tool usable in a method according to claim 14, wherein the tool comprises
- a guide tool with an alignment element to be aligned with a connector at one end of a tube,
- a measuring element, that is connected or connectable to the guide toll such that it can be shifted along the guide tool,
- wherein the measuring element has a circumferential marking line and an axial marking line.

16. Orienter for a method according to any of claims 10 to 13, having a contact edge and an inverted pyramid, the contact edge being annular, preferably circular and the inverted pyramid being tilted relative to the plane defined by the contact edge by a predetermined angle in a predetermined direction, wherein preferably the predetermined angle is not 0°

17. System comprising a prosthetic socket and at least a first orienter according to claim 14.

18. System according to claim 15, wherein the system further comprises at least a second orienter according to claim 14, wherein the first orienter is a socket orienter and the second orienter is a component orienter.

19. Method for manufacturing a system according to claims 17 or 18, the method comprising the following steps:
- a) Providing 3D-data of a prior inner surface of a prior socket and of its position and its orientation relative to a component adapter,
- b) Creating a 3D model of a prosthetic socket with a socket adapter,
wherein the prosthetic socket has an inner surface based on the scanned prior inner surface and an outer surface,
- c) Creating a 3D model of an orientation transmitter,
- d) Manufacturing of the prosthetic socket and the orientation transmitter based on the 3D models,
wherein the orientation transmitter is created such that the manufactured socket can be directly or indirectly connected to a component adapter by means of the orientation transmitter so that the position and orientation of the inner surface of the prosthetic socket relative to the position and orientation of the component adapter corresponds to the position and orientation of the prior inner surface of the prior socket to the component adapter.
